# EUROPEAN PATENT APPLICATION

(11) **EP 2 604 181 A1**
(43) Date of publication of application: **19.06.2013**
(21) Application number: 11816301.3
(22) Date of filing: 25.07.2011
(51) Int. Cl.: A61B 3/14

(54) **OPHTHALMOLOGIC IMAGING DEVICE**

(30) Priority: 10.08.2010 JP 2010179195
(71) Applicant: Kabushiki Kaisha Topcon, Tokyo 174-8580 (JP)
(72) Inventor: YAHAGI, Ryoichi, Tokyo 174-8580 (JP); OYAGI, Wataru, Tokyo 174-8580 (JP); OTSUKA, Hiroyuki, Tokyo 174-8580 (JP)
(74) Representative: Ilgart, Jean-Christophe
(86) International application number: PCT/JP2011/066828
(87) International publication number: WO 2012/020635

(57) **Abstract**

An ophthalmologic imaging device can shoot a diagnostically important area from the macula to the optic papilla in a wide angle of view without flares without trouble by a single imaging operation. An optical illumination system (25) includes a cornea aperture (34), an iris aperture (35), and a lens aperture (36) conjugate to the cornea (C) of the subject eye (E), the iris of the subject eye (E), and the posterior surface of the lens, respectively, and an split mark projection system (41) for focusing on the fundus (Ef) of the subject eye (E). A light source (37) is controlled by a controller to emit light for being able to obtain at least two consecutive fundus images. An inner aperture image (q3') corresponding to the lens aperture (36) is projected on the posterior surface of the lens. The controller (23) controls the lens aperture (36) so that for obtaining a second fundus image, the inner aperture image (q3') is projected at a position shifted relative to the optical axis of an optical observatory or imaging system (27, 26) from a position at which the aperture image is projected for obtaining a first fundus image.

## Description

### Technical Field

The present invention relates to an improvement in an ophthalmologic imaging device.

### Background Art

In related art a known ophthalmologic imaging device includes an imaging unit to capture an electronic image of a subject eye using apertures, an image processing unit to process the captured image of a fundus, and a selector unit to selectively switch the apertures (or iris aperture) disposed at different positions. This ophthalmologic imaging device can shoot the subject eye using the apertures at different positions by a single shutter operation to obtain a plurality of fundus images, and perform image processing on the fundus images so that the same parts of the subject eye in the obtained images overlap with each other (refer to Patent Document 1, for example). Also, it can acquire high-quality fundus images with flares removed.

Meanwhile, another known ophthalmologic imaging device aims to facilitate the focusing on a subject eye having a small pupil. For this purpose, it is configured to move a reference alignment position to an offset position on a monitor screen and offset the central axis of the pupil and the optical axis of a device body (refer to Patent Document 2, for example). According to this device, vignetting of a focus split due to the small pupil is prevented at the optical axis (reference position) of the device body and a split mark image on one side is projected onto the subject eye. Therefore, it is possible to focus the device body relative on the subject eye by placing the one-side split mark image in the center of a stick mirror image.

### Related Art Documents

### Patent Documents

Patent Document 1: Japanese Patent Application Publication No. 2009-285108
Patent Document 2: Japanese Patent Application Publication No. 2008-278914

### Disclosure of the Invention

### Problems to be Solved by the Invention

Another ophthalmologic imaging device comprises an illumination optical system to illuminate the fundus of a subject eye, having three apertures, a cornea aperture conjugate to the cornea, an iris aperture conjugate to the iris, and a lens aperture conjugate to the posterior surface of a lens. This type of device faces a problem in imaging a subject eye with a small pupil that the center portion of a captured fundus image tends to be dark due to a small iris.

To prevent the center portion of the captured fundus image from becoming dark, the device reduces the diameter of the lens aperture conjugate with the posterior surface of the lens to sufficiently illuminate the center of the fundus when imaging the fundus of the subject eye with a small pupil.

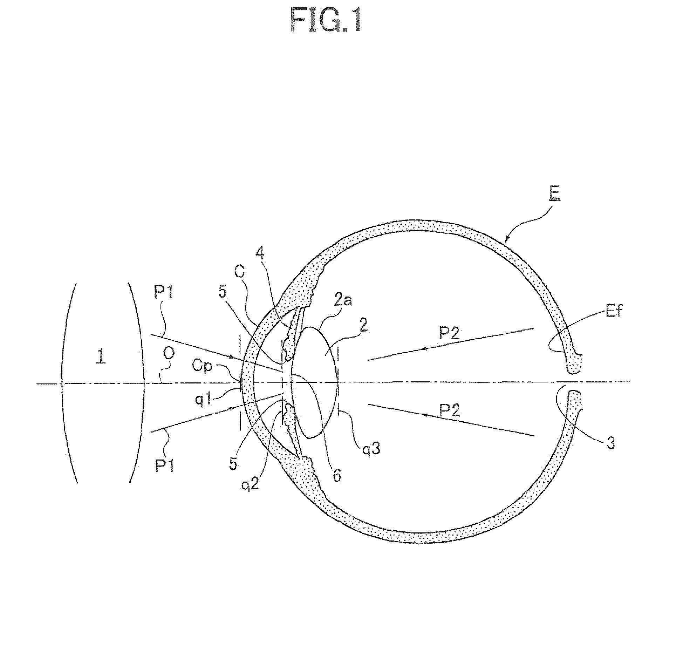

In the following the problem to be solved by the invention is described with reference to FIGs. 1 to 4. FIG. 1 shows a subject eye from which three aperture images are generated, and an objective lens 1, a lens aperture 2, a papilla 3, an iris 4, the inner margin of the iris 5, a pupil 6, a subject eye E, the cornea C of the subject eye E, the fundus Ef of the subject eye E, and the optical axis O of the objective lens 1 or the device body.

An illuminating ray P1 is guided to the subject eye E via the objective lens 1 and a cornea aperture image q1, an iris aperture image q2, a lens aperture image q3 are formed on the apex Cp of the cornea C and at positions approximately conjugate with the pupil 6 and the posterior surface 2a of the lens aperture 2, respectively. The illuminating ray P1 is incident on the subject eye E through the pupil 6 and illuminates the fundus Ef.

The illuminating ray P1 reflected by the fundus Ef is a reflected ray (observing ray or imaging ray) P2 to transmit through the center of the pupil 6 and return to the objective lens 1. The reflected ray P2 transmits through the objective lens 1 and is guided to the optical observation or imaging system for observation or imaging.

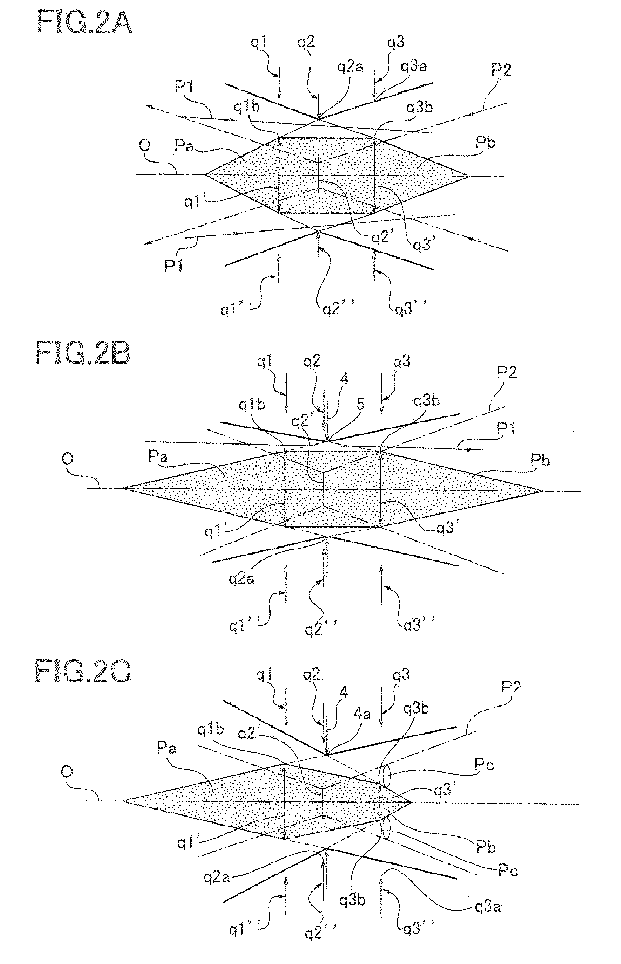

FIGs. 2A to 2C show the interaction among the illuminating ray P1, reflected ray P2, cornea aperture image q1, iris aperture image q2, and lens aperture image q3. The cornea aperture image q1 is made of an inner aperture image q1' and an outer aperture image q1". The iris aperture q2 is made of an inner aperture image q2' and an outer aperture image q2". The lens aperture image q3 is made of an inner aperture image q3' and an outer aperture image q3".

FIG. 2A shows the interaction among the illuminating ray P1, reflected ray P2 from the fundus Ef, cornea aperture q1, iris aperture image q2, and lens aperture image q3 when the subject eye E does not have a small pupil diameter. The illuminating ray P1 is restricted by the cornea aperture, iris aperture, and lens aperture, and turned to a ring-like beam, transmits through the pupil 6 to the inside of the subject eye E and illuminates the fundus Ef.

The cornea aperture image, iris aperture image, lens aperture image q1, q2, q3 work to prevent the occurrence of flares in the fundus image because scattered rays of the illuminating ray P1 reflected by the cornea C, iris 4, posterior surface 2a of the lens aperture 2 are incident on the objective lens 1, respectively.

The area of the pupil 6 on which the illuminating ray P1 is incident is mainly defined by the inner margin q2a of the outer aperture image q2", the outer margin q3b of the inner aperture image q3', the outer margin q1b of the inner aperture image q1' and the inner margin q3a of the outer aperture image q3". An area Pa is an area (non-incidence area) not to allow the illuminating ray P1 to transmit therethrough and an area Pb is an area (shadow area) in which the illuminating ray P1 incident through the pupil 6 cannot reach the fundus Ef.

The reflected ray P2 by the fundus Ef transmits through the shadow area Pb to the objective lens 1. The optical path of the reflected ray P2 is guided to an optical imaging system (not shown), not overlapping with that of the illuminating ray P1. Generally, the exit area of the reflected ray P2 is defined by the outer margin q3b of the inner aperture image q3' and the outer margin q1b of the inner aperture image q1'.

As shown in FIG. 2B, in case of a subject with a small pupil diameter, however, the incidence area of the illuminating ray P1 on the pupil 6 is defined by not the inner margin q3a of the outer aperture image q3" but the inner margin 5 of the iris 4 of the subject eye E. Because of this, the amount of illuminating ray P1 incident on the pupil 6 is decreased, extending the shadow area Pb so that an area that almost no illuminating ray P1 reaches occurs in the fundus Ef. As a result, a fundus image with a dark portion, for example, the macula will be obtained.

In view of this, to deal with a subject having a small pupil diameter in related art, the inner aperture of the lens aperture is replaced with one with a smaller diameter to reduce the size of the inner aperture image q3' formed on the posterior surface 2a of the lens aperture 2, as shown in FIG. 2C. This prevents the extension of the shadow area Pb toward the fundus Ef to secure the amount of the illuminating ray P1 incident on the subject eye E.

However, a problem arises when the size of the outer diameter of the inner aperture image q3' is decreased. As shown in FIG. 2C, a part Pc of the optical path of the reflected ray P2 overlaps with that of the illuminating ray P1. This may cause flares in a captured fundus image. To avoid the occurrence of flares, angle of view needs to be reduced. Thus, it is not possible to properly image a diagnostically important area from the macula to the optic papilla in a wide angle of view without flares without trouble by a single imaging operation.

Specially, in imaging with a xenon lamp, it requires a long time to charge the lamp so that consecutive images cannot be captured by a single imaging operation. This leads to placing a load on a subject temporally, psychologically.

Another solution may be moving the iris aperture. However, with the iris aperture moved, a fundus image may include parallax, which is troublesome for image synthesis.
Still another solution may be widening the pupil of the subject by a mydriatic agent. However, many of patients with a small pupil diameter suffer a diabetic glaucoma and the use of a mydriatic agent may worsen their glaucoma. It is therefore difficult to capture a diagnostically important area from the macula to the optic papilla in a wide angle of view.

An object of the present invention is to provide an ophthalmologic imaging device which can shoot a diagnostically important area from the macula to the optic papilla in a wide angle of view without flares without trouble by a single imaging operation.

### Means to Solve the Problems

An ophthalmologic imaging device according to the present invention includes an illumination optical system having a cornea aperture conjugate to a cornea of the subject eye, an iris aperture conjugate to an iris of the subject eye, a lens aperture conjugate to a posterior surface of a lens, and a split mark projection system to bring the fundus of the subject eye into focus. A light source is controlled by a controller to emit light for being able to obtain at least two consecutive fundus images. An inner aperture image corresponding to the lens aperture is projected onto the posterior surface of the lens. The controller is configured to control the lens aperture so that for obtaining a second fundus image, the aperture image is projected at a position shifted relative to an optical axis of the optical observatory or imaging system from a position at which the aperture image is projected for obtaining a first fundus image.

### The Effect of the Invention

According to the invention, it is possible to shoot a diagnostically important area from the macula to the optic papilla in a wide angle of view without flares without trouble by a single imaging operation.

### Brief Description of the Drawings

FIG. 1 shows the eyeball of a subject eye in which at least three aperture images are formed;
FIG. 2A is a view of the optical paths showing the relation among the illuminating ray and reflected ray from the fundus in FIG. 1 and aperture images in imaging a subject eye not having a small pupil diameter;
FIG. 2B is a view of the optical paths showing the relation among the illuminating rays, reflected ray from the fundus and aperture images in imaging the subject eye having a small pupil diameter, with an extended shadow area due to the small pupil diameter;
FIG. 2C shows the relation among the illuminating ray, reflected ray and aperture images in imaging the fundus of the subject eye having a small pupil diameter when a lens aperture of at least three apertures is replaced to reduce the shadow area;
FIG. 3 shows the exterior of an ophthalmologic imaging device according to one embodiment of the present invention;
FIG. 4 shows the optical systems of the ophthalmologic imaging device in FIG. 3;
FIG. 5 shows an example of observing a subject eye with a normal pupil diameter when the device body is not aligned with the subject eye and the fundus of the subject eye is not in focus;
FIG. 6 shows that the device body is aligned with the subject eye and the fundus of the subject eye is in focus;
FIG. 7 shows how the pupil diameter of the subject eye is measured on the basis of the image of an anterior eye of the subject;
FIG. 8 is a schematic plan view of the structure of a lens aperture;
FIG. 9 shows an example of how the fundus of a subject eye with a small pupil is seen;
FIG. 10 shows the subject eye with an alignment mark offset from the optical axis of an objective lens before the device body is aligned with the subject eye;
FIG. 11 is a flowchart showing a series of operations to shoot the fundus by the ophthalmologic imaging device according to one embodiment of the present invention;
FIG. 12 is a view of the optical paths showing the relation among the illuminating ray, at least three aperture images, and imaging ray when the subject eye has a small pupil diameter and the optical axis of the objective lens and the central axis of the pupil are offset from each other;
FIG. 13 shows the observation of a split mark image on the subject eye after the device body has completed the alignment with the subject eye;
FIG. 14 shows that a focus lens of the optical imaging system focuses on the fundus with a single split mark image in FIG. 13;

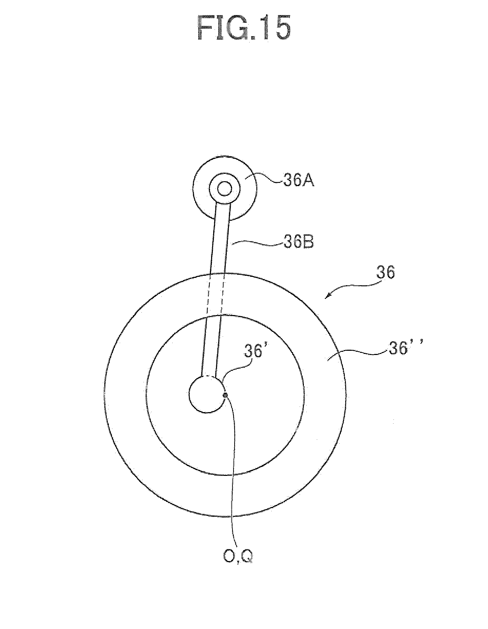

FIG. 15 is a plan view of the lens aperture in FIG. 8 when shifted;
FIG. 16A is a view of the optical paths showing the relation among the illuminating ray, aperture images, imaging ray and iris inner margin on the subject eye seen from the front when the lens aperture in FIG. 15 is shifted;
FIG. 16B is a horizontal cross section view of the optical paths in FIG. 16A, showing the relation among the illuminating ray, aperture images, imaging ray and iris inner margin when the lens aperture in FIG. 15 is shifted;
FIG. 17A is a view of the optical paths showing the relation among the illuminating ray, aperture images, imaging ray and iris inner margin on the subject eye seen from the front when the lens aperture in FIG. 15 is shifted to a symmetric position on the opposite side relative to the optical axis of the lens aperture;
FIG. 17B is a horizontal cross section view of the optical paths in FIG. 17A, showing the relation among the illuminating ray, aperture images, imaging ray and iris inner margin when the lens aperture in FIG. 15 is shifted to the symmetric position on the opposite side relative to the optical axis of the lens aperture;
FIG. 18A shows a fundus image captured by a first imaging;
FIG. 18B shows a fundus image captured by a second imaging;
FIG. 18C shows a synthetic image of the left half of the fundus image in FIG. 18A and the right half of the fundus image in FIG. 18B with no defects;
FIG. 19A is a view of the optical paths showing the relation among the illuminating ray, aperture images, imaging ray and iris inner margin on the subject eye seen from the front when the lens aperture is shifted in advance to one side for observation and then, the subject eye is continuously shot;
FIG. 19B is a horizontal cross section view of the optical paths in FIG. 19A when the lens aperture is shifted in advance to one side for observation and then, the subject eye is continuously shot;
FIG. 19C is a view of the optical paths showing the relation among the illuminating ray, aperture images, imaging ray and iris inner margin on the subject eye seen from the front when the lens aperture is shifted in advance to a symmetric position on the opposite side relative to the optical axis of the lens aperture for observation and then, the subject eye is continuously shot;
FIG. 19D is a horizontal cross section view of the optical paths in FIG. 19C when the lens aperture is shifted in advance to one side for observation and then the subject eye is continuously shot;
FIG. 20 is a view of the optical paths showing the relation among the illuminating ray, aperture images, imaging ray and iris inner margin on the subject eye seen from the front when the lens aperture is shifted at high speed;
FIG. 21A is a view of the optical paths showing the relation among the illuminating ray, reflected ray, aperture images and iris inner margin when the lens aperture is moved away from the optical path of the illumination optical system for observation and it is inserted thereinto for imaging;
FIG. 21B is a view of the optical paths showing the relation among the illuminating ray, aperture images at a shift position on one side, reflected ray, and iris inner margin when the lens aperture is moved away from the optical path of the illumination optical system for observation and it is inserted thereinto for imaging the subject eye;
FIG. 21C is a view of the optical paths showing the relation among the illuminating ray, aperture images in FIG. 21B shifted to the other side, reflected ray, and iris inner margin when the lens aperture is moved away from the optical path of the illumination optical system for observation and it is inserted thereinto for imaging;
FIG. 22A is a view of the optical paths showing the relation among the illuminating ray, at least three aperture images, and imaging ray when the optical axis of the optical imaging system and the pupil central axis are offset at imaging, as in FIG. 12;
FIG. 22B is a view of the optical paths showing the relation among the illuminating ray, imaging ray, aperture images, and iris inner margin in FIG. 22A on the subj ect eye seen from the front when the optical axis of the optical imaging system and the pupil central axis are offset at imaging;
FIG. 22C is a view of the optical paths showing the relation among the illuminating ray, at least three aperture images and imaging ray when at imaging the lens aperture is shifted with the optical axis of the optical imaging system and the pupil central axis offset;
FIG. 22D is a view of the optical paths showing the relation among the illuminating ray, imaging ray, aperture images, and iris inner margin in FIG. 22C on the subject eye seen from the front when at imaging the optical axis of the optical imaging system and the pupil central axis are offset;
FIG. 23 shows the other optical systems of the fundus camera in FIG. 3 and another structure of the three apertures of the illumination optical system.

### Description of the Embodiments

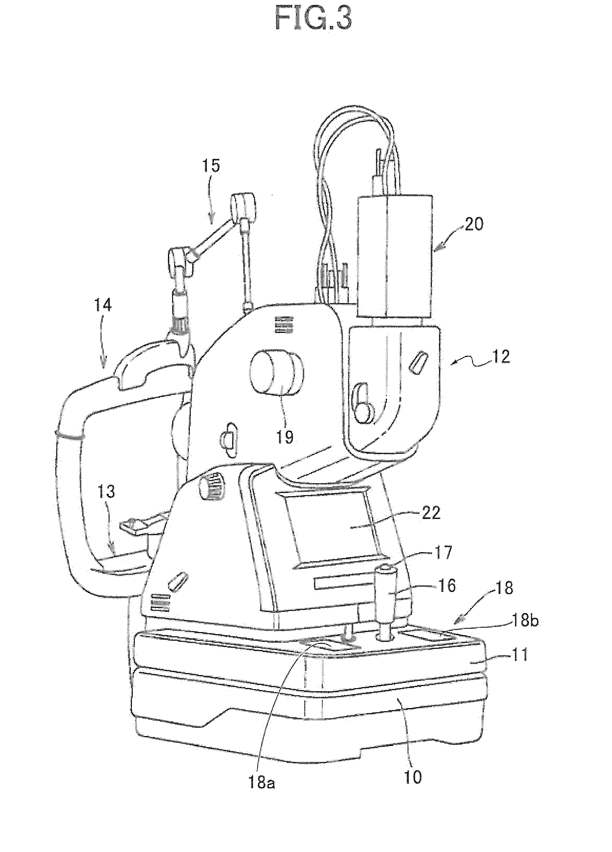

FIG. 3 shows the exterior of a non-mydriatic fundus camera according to the present invention. In the drawing it includes a base 10, a mount 11, a device body 12, a jaw receiver 13, a forehead pad 14, an external fixation lamp 15, a joystick 16, a shooting switch 17, an operation panel 18, a focus handle 19, and a TV camera 20 as a photographic camera. For the sake of explanation, a subject eye E not having a small pupil diameter is described first.

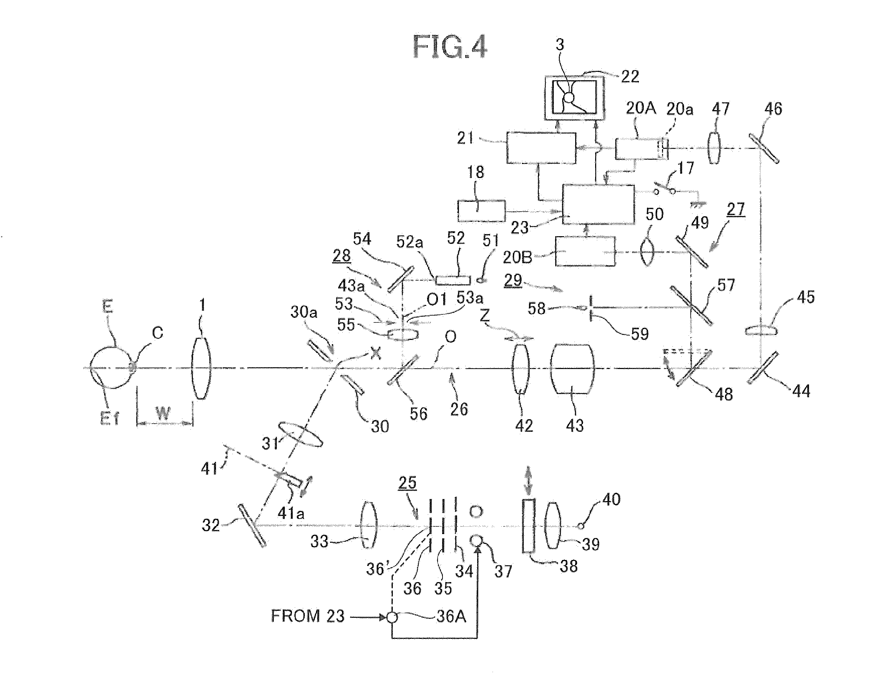

In FIG. 4 the TV camera 20 includes an imaging TV camera 20A and an observation TV camera 20B. The imaging TV camera 20A is connected to a monitor 22 and a controller 23 via a still video recorder 21. The observation TV camera 20B is connected to the monitor 22 via the controller 23.

The device body 12 in FIG. 4 includes an illumination optical system 25 to illuminate the fundus Ef of a subject eye E, an optical imaging system 26 to shoot the fundus Ef, an optical observation system 27 to observe the fundus Ef, an alignment optical system 28 to align the device body 12 with the subject eye E, and an internal fixation mark projection system 29 to projecting a fixation mark onto the fundus Ef to fix the subject eye E.

The illumination optical system 25 irradiates the fundus Ef with infrared light for observation and irradiates it with visible light for imaging. The illumination optical system 25 includes an objective lens 1, a hole mirror 30, a relay lens 31, a reflection mirror 32, a relay lens 33, a cornea aperture 34 approximately conjugate to the cornea C of the subject eye E, an iris aperture 35 approximately conjugate to the iris 4 (pupil 6) of the subject eye E, a lens aperture 36 approximately conjugate to the posterior surface 2a of the lens 2, an xenon lamp 37 as light source for imaging, an IR filter 38, a condenser lens 39 and a halogen lamp 40 as light source for observation. The hole mirror 30 becomes conjugated with the cornea C of the subject eye E when the distance between the subject eye E and the objective lens 1 is set to an appropriate operation distance.

A stick mirror 41a constitutes a part of a split (focus) mark projection system 41 and is detachably inserted into the optical path of the illumination optical system 25 to be able to conjugate with the fundus Ef of the subject eye E (refer to Japanese Patent Application Publication No. H9-66032 for structural details).

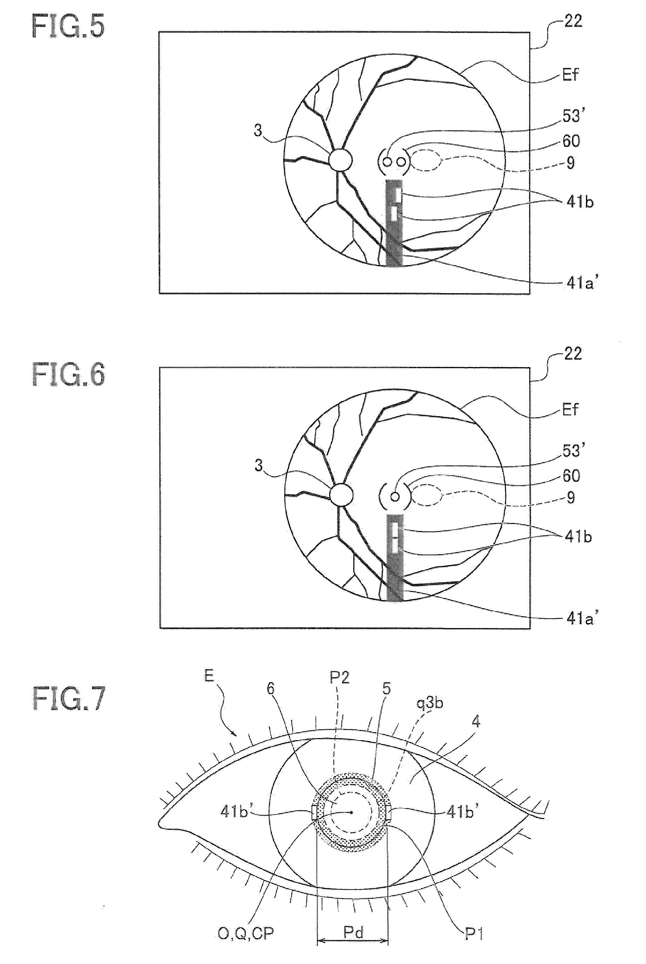

The split mark projection system 41 moves along the optical axis of the illumination optical system 25 together with the optical observation system 27 and a focus lens 42 of the optical imaging system 26, to allow a not-shown split mark plate and the fundus Ef to optically conjugate with each other constantly. If the fundus Ef and split mark plate are not conjugate, two split mark images 41b, 41b are separately seen horizontally in FIG. 5. The subject eye is brought in focus by aligning the split mark images. FIG. 5 shows the macula 9 of the fundus Ef.

Thus, the split mark images 41b, 41b by the split mark projection system 41 are displayed on the screen of the monitor 22, and when the split mark images 41b match each other, the subject eye is determined to be in focus and an imaging operation follows in general.

The optical imaging system 26 captures a still image of the fundus Ef illuminated by the illumination optical system 25. It includes the objective lens 1, hole mirror 30, focus lens 42, an imaging lens 43, a reflection mirror 44, a field lens 45, a reflection mirror 46, a relay lens 47, and the imaging TV camera 20A. The imaging TV camera 20A is optically conjugated with the fundus Ef.

The optical observation system 27 observes the fundus Ef illuminated by the illumination optical system 25. It is branched from the optical path of the optical imaging system 26 by a quick return mirror 48. It includes a reflection mirror 49, a relay lens 50, and the observation TV camera 20B and is disposed at a position conjugate with an image sensor 20a relative to the quick return mirror 48.

The alignment mark projector 28 projects an alignment mark onto the subject eye E, and includes an LED 51 as alignment light source, an optical guide 52 to guide the light from the LED 51, a reflection mirror 54 to reflect the light from the optical guide 52 to a double-hole aperture 53, a relay lens 55, a half mirror 56 to branch from the optical imaging system 26, a hole mirror 30, and the objective lens 1. The double-hole aperture 53 functions to project an alignment ray to the subject eye E. When the operation distance W is not proper, an alignment image 53' based on the alignment ray is separately projected onto the subject eye E.

The alignment ray is emitted from an exit end 52a of the optical guide 52 and reflected by the reflection mirror 54 to the double-hole aperture 53. Then, it is guided to the relay lens 55 through the hole 53a of the double-hole aperture 53.

The alignment ray transmits through the relay lens 55 and is reflected by the half mirror 56 to the hole mirror 30. The relay lens 55 forms an intermediate image from the exit end 52a (alignment mark 53') of the optical guide 52 at the center X of the hole 30a of the hole mirror 30, as shown in FIG. 4. A pair of alignment rays forming the alignment mark at the center X of the hole 30a is guided to the cornea C of the subject eye E via the objective lens 1.

The inner fixation mark projection system 29 is branched from the optical path of the optical observation system 27 by a dichroic mirror 57. The dichroic mirror 57 has property to allow infrared light to transmit therethrough and reflect visual light. The inner fixation mark projection system 29 projects a fixation mark onto the subject eye E, and includes a fixation light source 58, a mask plate 59, and the dichroic mirror 57. Thereby, the fixation mark is presented to the subject eye E.

In general an alignment mark (parenthesis) 60 is displayed at a reference position of the monitor 22. The reference position refers to the center of the fundus Ef or the center of the optical axis O. The alignment mark 60 is controlled by the controller 23.

The operation panel 18 in FIG. 3 includes the joystick 16 at the center, a left side operation panel 18a, and a right side operation panel 18b. These panels are provided with various buttons. The controller 23 presents the alignment mark 60 at the reference position in conjunction with a manipulation of the buttons.

When the operation distance W is set properly relative to the subject eye E and relative to the device body 12 vertically, horizontally, the alignment mark image 53' matches the alignment mark 60 at the center, as shown in FIG. 6.

In manual operation, by manipulation of the focus handle 19, the split mark images 41b are moved to match each other and the focus lens 42 is moved to focus on the fundus Ef. Then the fundus in focus is shot with the shooting switch 17.

A non-mydriatic fundus camera can generally observe the image of an eye to measure a pupil diameter. The controller 23 can measure a pupil diameter Pd on the basis of an anterior eye image as shown in FIG. 7. A pupil diameter of 3.0 to 3.5 mm or less is typically referred to as small pupil diameter. The controller 23 determines that the subject eye E is a small pupil when the pupil diameter Pd is 3.5 mm or less. FIG. 7 shows the subject eye E with a small pupil in which split marks 41 b' are vignetted.

In the following embodiments in which a small pupil is shot are described.

### First Embodiment

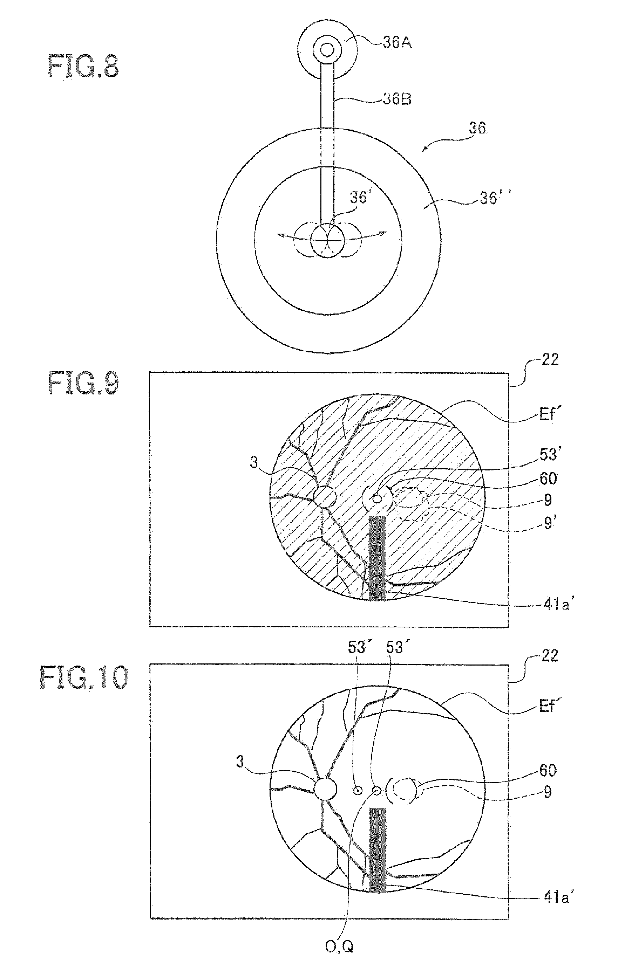

In the present embodiment an inner aperture 36' of the lens aperture 36 is horizontally swung by imaging operation in FIG. 8. In FIG. 8 the lens aperture 36 includes an outer aperture 36", a drive motor 36A for the inner aperture 36', a joint arm 36B to join the drive motor 36A and inner aperture 36'.

Along with the horizontal movement of the inner aperture 36', a corresponding inner aperture image q3' is projected onto the posterior surface 2a of the lens aperture 2 at a horizontally shifted position relative to the optical axis O. As previously described, if the subject eye E has a small pupil, the illuminating ray P1 is vignetted by near the inner margin 5 of the iris 4 in FIG. 7 so that the entire fundus image Ef' is darkened on the monitor 22. Further, the split marks 41b', 41b' in FIG. 7 are also vignetted and disappear from a stick mirror image 41 a' as shown in FIG. 9. Thus, the subject eye E cannot be focused. Moreover, a shadow area 9' occurs in the vicinity of the macula 9 of the fundus Ef. Note that hatching in FIG. 9 represents that the entire fundus image is dark.

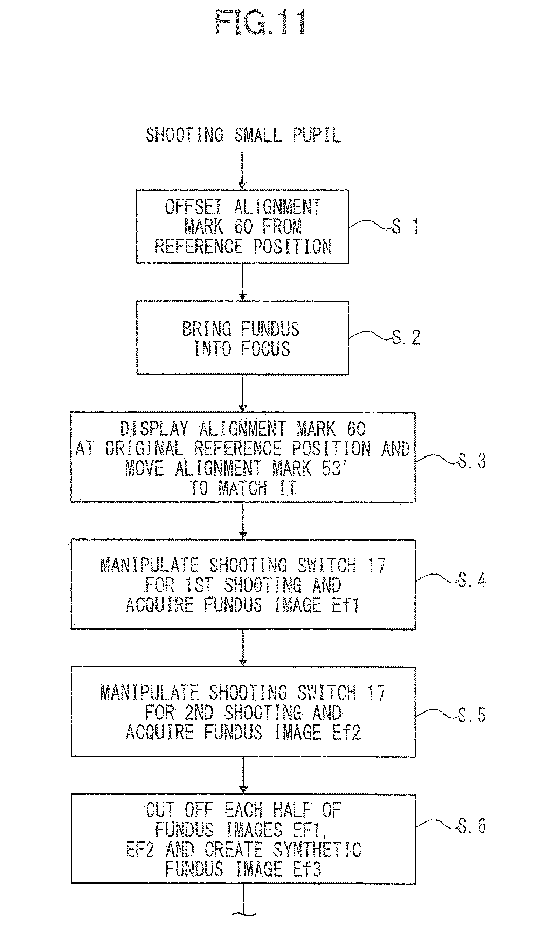

In view of this, in FIG. 10 the alignment mark 60 is shifted to be offset from the reference position in FIG. 9 to have the cornea apex Cp (pupil central axis Q) of the subject eye E offset from the optical axis O of the objective lens 1 (S. 1 in FIG. 11).

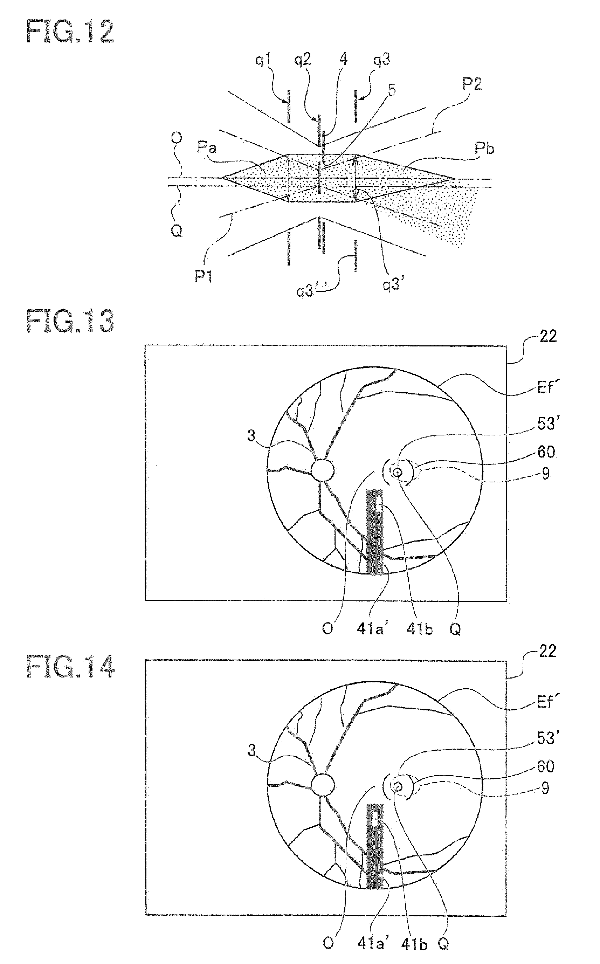

Here, the device body 12 is operated to place the alignment mark 53' in the parenthesis of the alignment mark 60 to match the mark 60. Then, the optical axis O of the optical imaging system 26 is offset from the pupil central axis Q of the subject eye E in FIG. 12. Accordingly, the illuminating ray P1 and split marks are incident on the subject eye E from one side, and one split mark image 41b is observed as shown in FIG. 13. If the optical imaging system 26 is not focusing on the fundus Ef in FIG. 13, the split mark image 41b appears at a position shifted from the center of the stick mirror image 41a'.

Then, by manipulation of the focus handle 19, the split mark image 41b is moved so that the center thereof along the width coincides with the center of the stick mirror image 41a' (S. 2), as shown in FIG. 14. This completes the focusing of the optical imaging system 26 on the fundus Ef.

Then, in FIG. 9 the alignment mark 60 is returned to the original reference position, and by manipulation of the device body 12, the alignment mark 53' is placed in the parenthesis of the alignment mark 60 to match it (S.3). Thereby, the imaging optical axis O and pupil central axis Q coincide with each other again.

By manipulation of the shooting switch 17, the controller 23 controls the drive motor 36A to drive to swing the inner aperture 36' to a left-side predetermined position. Simultaneously, the controller 23 controls the xenon lamp 37 to emit light.

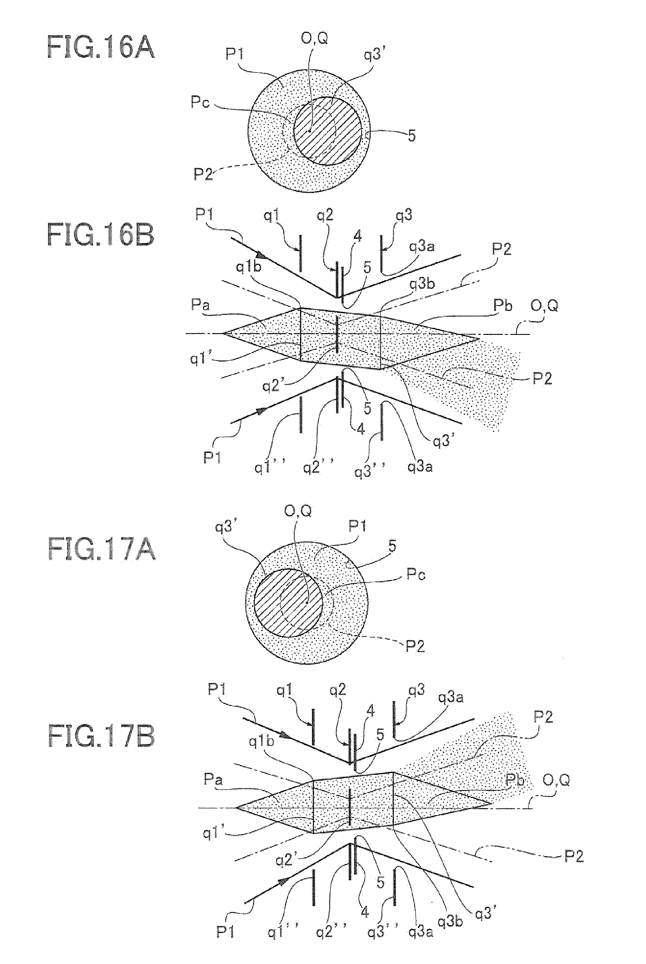

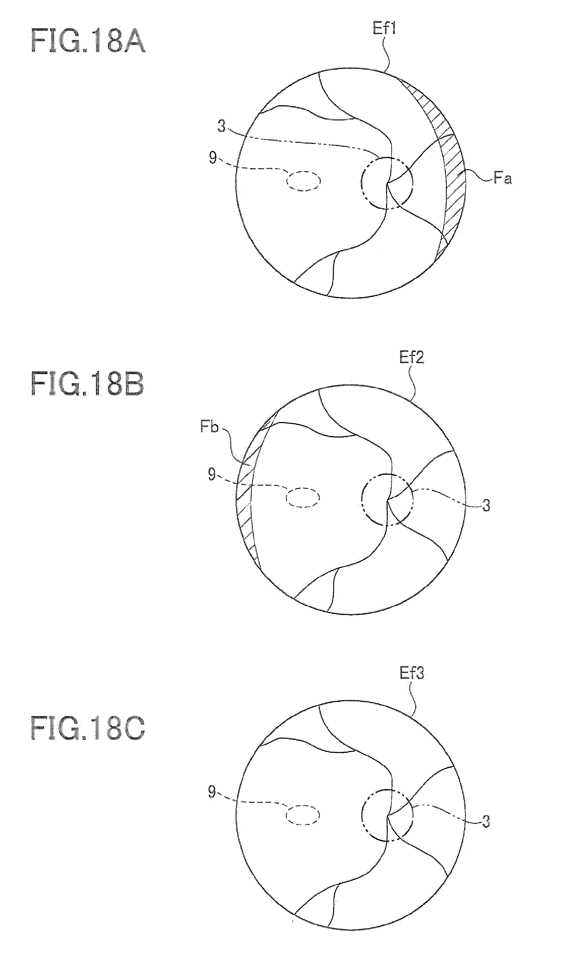

In accordance with the leftward movement of the inner aperture 36', the inner aperture image q3' is shifted to the right side, and the amount of the illuminating light P1 incident on the subject eye E from one side is increased, as shown in FIGs. 16A, 16B. Thereby, a fundus image Ef1 in FIG. 18A is acquired and temporarily stored in a storage medium of the controller 23 (S. 4).

The controller 23 then moves the inner aperture 36' to a symmetric, right-side position relative to the optical axis O and simultaneously controls the xenon lamp 37 to emit light. In accordance with the rightward movement of the inner aperture 36', the inner aperture image q3' is shifted rightward as shown in FIGs. 17A, 17B, which increases the amount of the illuminating light P1 incident on the subject eye E from one side. Thereby, a fundus image Ef2 in FIG. 18B is acquired (S. 5) and temporarily stored in the storage medium of the controller 23.

Thus, by a single imaging operation, the inner aperture image q3' is shifted in opposite directions to acquire the two fundus images Ef1, Ef2, as shown in FIGs. 18A, 18B. When the inner aperture image q3' is shifted rightward, the optical path of the illuminating ray P1 and that Pc of the imaging ray P2 partially overlap with each other in FIG. 18A. Because of this, a flare F1 occurs around the right side of the fundus image Ef1 in FIG. 18A. When the inner aperture image q3' is shifted leftward, the optical path of the illuminating ray P1 and that Pc of the imaging ray P2 partially overlap with each other. Because of this, a flare Fb occurs around the left side of the fundus image in FIG. 18B.

Meanwhile, since the illuminating ray P1 is incident only from one side, the fundus image Ef1 in FIG. 18A appears dark slightly from right to left, and the fundus image Ef2 in FIG. 18B appears dark slightly from left to right. However, the occurrence of the shadow area 9' (FIG. 9) in both the fundus images Ef1, Ef2 is prevented.

The controller 23 cuts off a left half of the fundus image Ef1 in FIG. 18A and a right half of the fundus image Ef2 to form a synthetic fundus image Ef3 in FIG. 18C. The synthetic fundus image Ef3 with no shadow area and flares removed is stored in the storage medium (S.6).

In the first embodiment the lens aperture 36 is mechanically moved. However, the cornea aperture 34, iris aperture 35, lens aperture 36 can be configured of a liquid crystal display plate (not shown). In this case the inner aperture image q3' can be projected on the posterior surface 2a of the lens aperture 2 at a shift position by electro-optically changing the position of the inner aperture of the liquid crystal display plate corresponding to the inner aperture 36' of the lens aperture 36.

### Second Embodiment

In the first embodiment, for observation the inner aperture image q3' associated with the inner aperture 36' is placed at the center of the optical axis O of the objective lens 1. For imaging, the inner aperture image q3' is shifted to horizontal, symmetric positions from the center.
Meanwhile, in the second embodiment, for observation the subject eye is brought in alignment while the inner aperture image q3' is shifted in advance to either side of the optical axis O of the objective lens 1 and the alignment mark 60 is displayed at the reference position. Then, the alignment mark 60 is moved from the reference position to an offset position and the subject eye is focused. The observation of the fundus Ef can be improved from the first embodiment by shifting the inner aperture image q3' in advance to either side of the optical axis O of the objective lens 1.

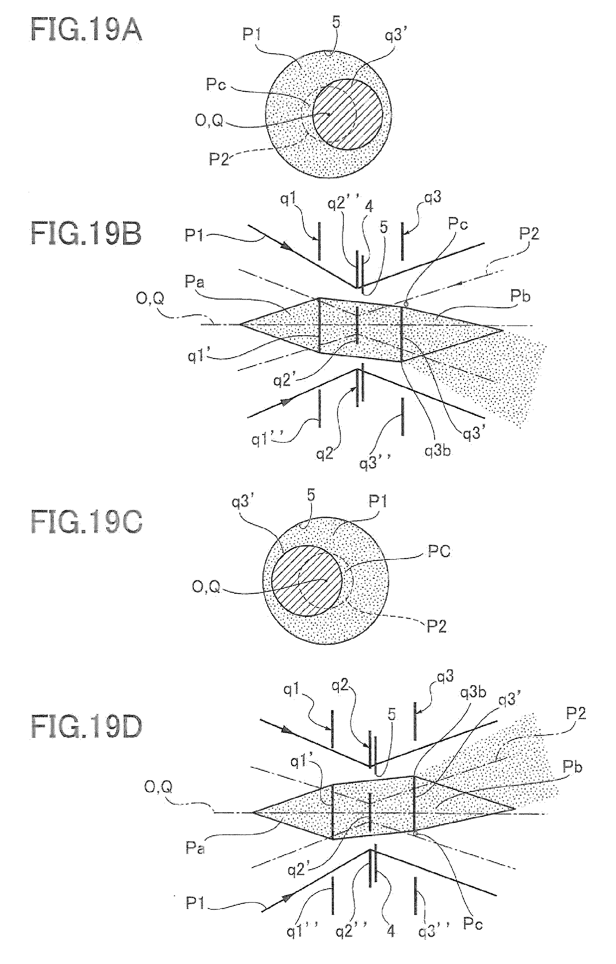

For example, in FIG. 19A the inner aperture image q3' is projected on the right-side of the posterior surface 2a of the lens aperture 2 disproportionately relative to the optical axis O. First, the alignment mark 60 is offset from the reference position in FIG. 10 (S.1 in FIG. 11). By manipulation of the device body 12, the alignment mark 53' is placed in the parenthesis of the alignment mark 60, and by manipulation of the focus handle 19, the split mark image 41b is moved so that the center thereof along the width coincides with the center of the stick mirror image 41 a' (S. 2 in FIG. 11).

Next, the alignment mark 60 is returned to the original reference position in FIG. 9. By manipulation of the device body 12, the alignment mark 53' is placed in the parenthesis of the alignment mark 60 to match therewith (S.3).

Then, the xenon lamp 37 is controlled to emit light concurrently with the manipulation of the shooting switch 17. A first fundus image is captured while the relation between the illuminating ray P1 and the inner aperture image q3' as in FIG. 19B is maintained. Consecutively, the inner aperture 36' is shifted to the other position to project the inner aperture image q3' on the symmetric position relative to the optical axis O in FIG. 19C. A second fundus image is then captured while the relation between the illuminating ray P1 and the inner aperture image q3' is maintained as in FIG. 19D.

As configured above, the inner aperture 36' is placed at a shift position relative to the optical axis O of the objective lens before observation, so that it is possible to shorten the time taken for moving the inner aperture 36' and continuously shoot the fundus in a shorter time.

### Third Embodiment

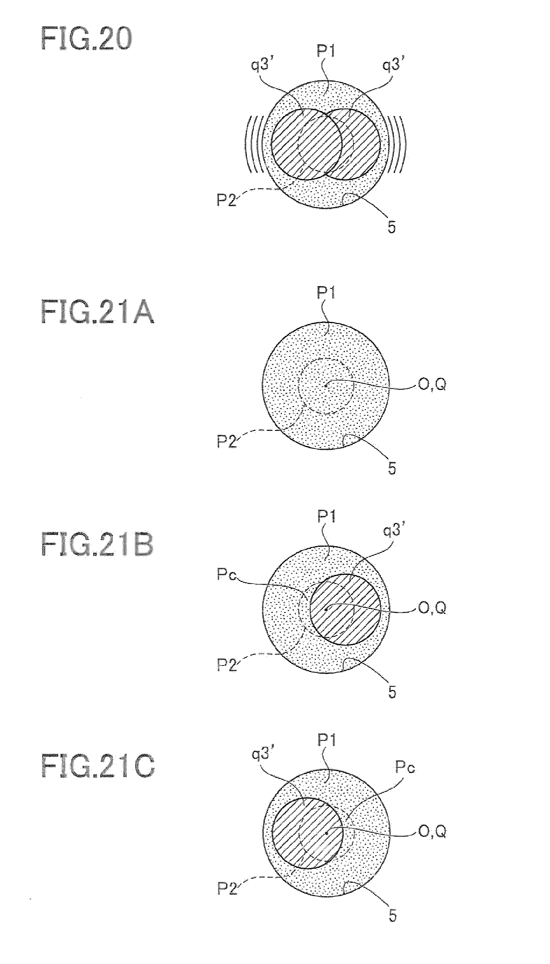

In the third embodiment the inner aperture 36' is horizontally shifted at high speed during the observation of the fundus. By swinging the inner aperture 36' at high speed, the inner aperture image q3' is quickly shifted horizontally as shown in FIG. 20 so that it is possible to observe even the fundus with a small pupil at a balanced light amount close to that for a captured image. The rest of the operations is the same as in the first embodiment.

A swing cycle of the inner aperture 36' is preferably synchronized with the timing at which an image signal of the observation camera 20B is acquired. In synchronization with the manipulation of the shooting switch 17, the inner aperture 36' is swung to acquire the two consecutive fundus images Ef1, Ef2. Thus, a fundus image close to the synthetic fundus image Ef3 can be pre-checked.

### Fourth Embodiment

In observation the subject eye is brought in alignment while the fundus is being observed with the inner aperture 36' away from the optical path of the illuminating ray P1 as shown in FIG. 21A and the alignment mark 60 displayed from the beginning at an offset position from the reference position. According to the fourth embodiment, since the inner aperture 36' is moved away during observation, it is possible to prevent the occurrence of the shadow area 9' about the center of the fundus Ef due to a vignetting of the illuminating ray P1 by the iris and properly observe the fundus Ef. Also, it is possible to prevent a vignetting of the split mark 41b' near the inner margin of the iris 5.

Then, after the focus operation, the alignment mark 60 is displayed at the reference position for re-alignment of the subject eye and the shooting switch 17 is manipulated. Concurrently with the manipulation of the shooting switch 17, the inner aperture 36' is inserted into the optical path, and the inner aperture image q3' is projected at a shift position as shown in FIG. 21B. At the same time light is emitted from the xenon lamp 37 to shoot the fundus first time.
Subsequently, the inner aperture 36' is shifted to the other position to project the inner aperture image q3' at a shift position as shown in FIG. 21C and shoot the fundus second time. According to the fourth embodiment, it is possible to shorten the time taken for displaying the alignment mark 60 at the reference position for aligning the subject eye in the first embodiment.

### Fifth Embodiment

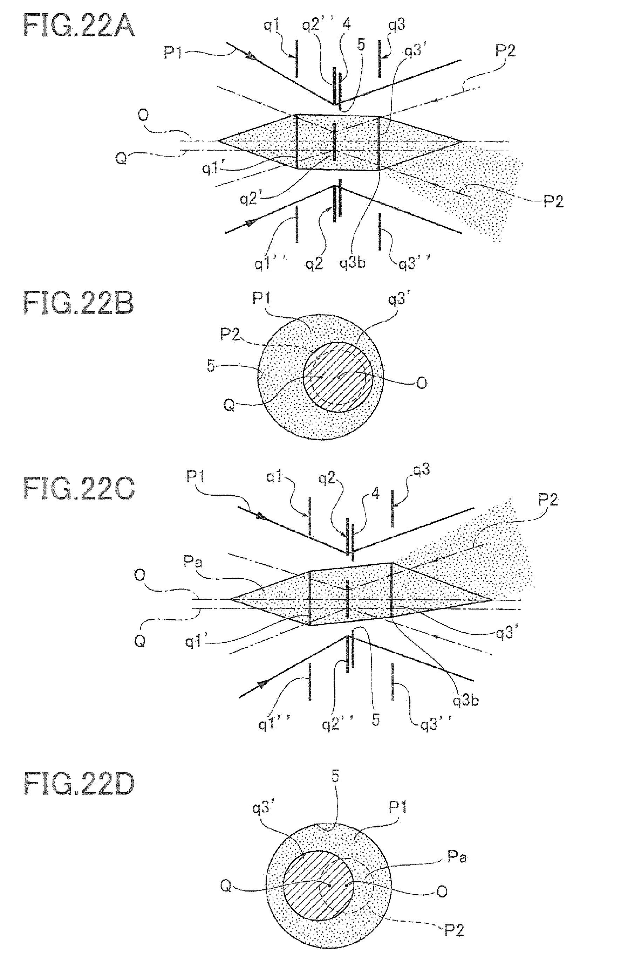

In the fifth embodiment the alignment mark 60 is displayed on the screen of the monitor 22 at a shift position from the reference position from the beginning, as shown in FIG. 10. By adjusting the device body 12, the alignment mark 53' is positioned in the alignment mark 60, and the pupil central axis Q and the optical axis O of the device body 12 are set at predetermined offset positions in FIG. 22A.

Here, the one split mark image 41b is adjusted by focus operation to be positioned at the center of the stick mirror image 41a' in FIG. 14. Then, a first fundus image is captured by manipulation of the shooting switch 17. During imaging the inner aperture 36' is not moved so that the optical path of the illuminating ray P1 is prevented from overlapping with that of the imaging ray P2 as shown in FIG. 22B. Thus, the fundus image free from flares can be obtained at a first imaging.

Then, the inner aperture 36' is moved to shift the inner aperture image q3' as in FIGs. 22C, 22D. By light emission from the xenon lamp 37, a second fundus image is obtained. According to the fifth embodiment at the first imaging the inner aperture 36' does not need to be shifted. Accordingly, it is possible to reduce the imaging time. Further, the re-alignment operation is not necessary, therefore, alignment time can be shortened accordingly.

### Sixth Embodiment

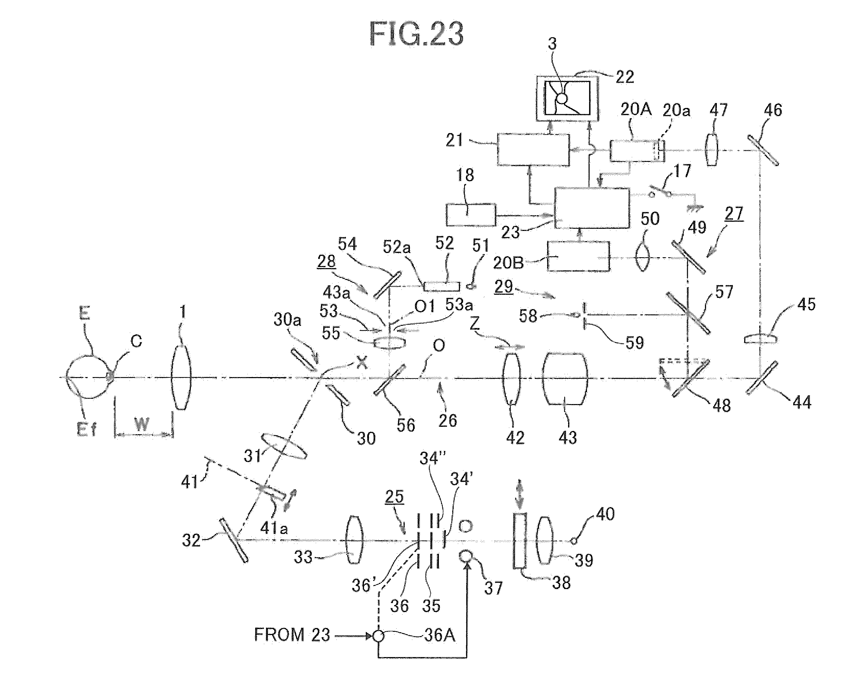

FIG. 23 shows the other optical systems of the fundus camera in FIG. 3 and another structure of the three apertures of the illumination optical system. In the present embodiment at least three apertures of the illumination optical system 25 are a light shielding plate 34' approximately conjugate to the cornea C of the subject eye E, an iris aperture 35 approximately conjugate to the iris 4 of the subject eye E, a lens aperture 36 approximately conjugate to the posterior surface 2a of the lens 2 of the subject eye E and an aperture diaphragm 34". The aperture diaphragm 34" is provided between the iris aperture 35 and the light shielding plate 34' and works to equalize the illuminance of the fundus Ef (refer to Japanese Patent Publication No. S62-16092).
Note that the number of iris apertures 35 can be two or more instead of one. Further, the lens aperture 36 can be comprised of light shielding plates corresponding to the inner and outer apertures 36', 36" and the one corresponding to the outer aperture 36" is disposed between the one corresponding to the inner aperture 36' and the iris aperture 35. The light shielding plate corresponding to the inner aperture 36' can be moved horizontally.

### Another Example

In addition to the above embodiments, the apertures can be a cornea aperture conjugate to the cornea and a black plate (not shown) conjugate to the anterior surface of the lens aperture (refer to Japanese Patent Publication No. S62-16092). The black plate can be moved horizontally.

### CROSS REFERENCE TO RELATED APPLICATION

The present application is based on and claims priority from Japanese Patent Application No. 2010-179195, filed on August 10, 2010, the disclosure of which is hereby incorporated by reference in its entirety.

### Description of Numeral Codes

23 controller
25 illumination optical system
26 optical imaging system
27 optical observation system
34 cornea aperture
35 iris aperture
36 lens aperture
37 LED (light source)
41 split mark projection system
E subject eye
C cornea
Ef fundus
q3' inner aperture image

## Claims

1. An ophthalmologic imaging device comprising:
an illumination optical system to illuminate a fundus of a subject eye, comprising three apertures and a light source, the at least three apertures being a cornea aperture approximately conjugate to a cornea of the subject eye, an iris aperture approximately conjugate to an iris of the subject eye, and a lens aperture approximately conjugate to a posterior surface of a lens to project an aperture image onto the posterior surface, the light source being controlled to emit light for being able to obtain at least two consecutive fundus images;
a split mark projection system to bring the fundus of the subject eye into focus;
an optical observatory or imaging system including a photographic camera to observe the fundus; and
a controller to control the light source and the photographic camera as well as to control the lens aperture so that for obtaining a second fundus image, the aperture image is projected at a position shifted relative to an optical axis of the optical observatory or imaging system from a position at which the aperture image is projected for obtaining a first fundus image.

2. An ophthalmologic imaging device according to claim 1, wherein
the controller is configured to control the lens aperture so that for obtaining the first fundus image, the aperture image is projected at a shift position on one side relative to the optical axis and for obtaining the second fundus image, the aperture image is projected at a symmetric shift position on the other side.

3. An ophthalmologic imaging device according to claim 2, wherein
for observing the fundus before obtaining the first fundus image, the aperture image is projected in advance at the shift position on one side relative to the optical axis.

4. An ophthalmologic imaging device according to claim 1, wherein
the controller is configured to control the lens aperture so that for obtaining the first fundus image, a central axis of a pupil of the subj ect eye is offset from the optical axis and the aperture image is set to be coaxial with the optical axis, and for obtaining the second fundus image, the aperture image is shifted from the optical axis.

5. An ophthalmologic imaging device according to claim 2, wherein
the controller is configured to move the lens aperture away from an optical path of the illumination optical system for observing the fundus.

6. An ophthalmologic imaging device according to claim 1, wherein the controller is configured to:
for observing the fundus, control the lens aperture so that the aperture image can be reciprocatively, at a high speed, projected onto shift positions symmetric to the optical axis, and
for imaging the fundus, control the light source to emit light in synchronization with the shifting of the positions of the aperture image.
